# EUROPÄISCHE PATENTSCHRIFT

(11) **EP 2 453 986 B1**
(45) Veröffentlichungstag und Bekanntmachung des Hinweises auf die Patenterteilung: **03.06.2015**
(21) Anmeldenummer: 10737792.1
(22) Anmeldetag: 22.06.2010
(51) Int. Cl.: A61N 5/10

(54) **VERFAHREN ZUR BESTRAHLUNG EINES SICH BEWEGENDEN ZIELVOLUMENS SOWIE BESTRAHLUNGSANLAGE**
METHOD FOR IRRADIATION A MOVING TARGET VOLUME, AND IRRADIATION SYSTEM
PROCÉDÉ D'IRRADIATION D'UN VOLUME CIBLE EN MOUVEMENT ET INSTALLATION D'IRRADIATION

(30) Priorität: 15.07.2009 DE 102009033318
(43) Veröffentlichungstag der Anmeldung: 23.05.2012
(73) Patentinhaber: GSI Helmholtzzentrum für Schwerionenforschung GmbH, 64291 Darmstadt (DE)
(72) Erfinder: BERT, Christoph, 63741 Aschaffenburg (DE); RIETZEL, Eike, 64331 Weiterstadt (DE)
(74) Vertreter: Mergel, Volker
(86) Internationale Anmeldenummer: PCT/EP2010/058769
(87) Internationale Veröffentlichungsnummer: WO 2011/006737

(56) Entgegenhaltungen:
- DE-A1-102007 014 715
- US-A1- 2009 095 921
- BERT CHRISTOPH ET AL: "4D treatment planning for scanned ion beams" RADIATION ONCOLOGY, BIOMED CENTRAL LTD, LO LNKD- DOI:10.1186/1748-717X-2-24, Bd. 2, Nr. 1, 3. Juli 2007 (2007-07-03), Seite 24, XP021030775 ISSN: 1748-717X
- PARODI K ET AL: "First 4D in-beam PET measurement for beam tracking of a moving phantom with a scanned carbon ion beam" NUCLEAR SCIENCE SYMPOSIUM CONFERENCE RECORD, 2008. NSS '08. IEEE (19-25 OCT. 2008), IEEE, PISCATAWAY, NJ, USA, 19. Oktober 2008 (2008-10-19), Seiten 4520-4524, XP031418564 ISBN: 978-1-4244-2714-7
- PHILLIPS M H ET AL: "Effects of respiratory motion on dose uniformity with a charged particle scanning method" PHYSICS IN MEDICINE AND BIOLOGY, TAYLOR AND FRANCIS LTD. LONDON, GB LNKD- DOI:10.1088/0031-9155/37/1/016, Bd. 37, Nr. 1, 1. Januar 1992 (1992-01-01) , Seiten 223-233, XP020021933 ISSN: 0031-9155
- GRÖZINGER SVEN OLIVER: "Volume conformal irradiation of moving target volumes with scanned ion beams" INTERNET CITATION 12. Februar 2004 (2004-02-12), Seiten 1-3,I, XP002595362 Gefunden im Internet: URL:http://tuprints.ulb.tu-darmstadt.de/40 7/ [gefunden am 2010-08-05]

## Beschreibung

Die Erfindung betrifft ein Verfahren zur Bestrahlung eines sich bewegenden Zielvolumens bzw. eine Bestrahlungsanlage zur Durchführung eines derartigen Verfahrens. Ein derartiges Verfahren wird insbesondere eingesetzt bei einer tatsächlichen und/oder simulierten Bestrahlung eines sich bewegenden Zielvolumens zur genauen Dosisdeposition im Zielvolumen.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nicht-therapeutischen Gebieten Anwendung. Hierzu gehören beispielsweise Forschungsarbeiten, etwa zur Produktentwicklung, im Rahmen der Partikeltherapie, die an nicht-lebenden Phantomen oder Körpern durchgeführt werden, Bestrahlungen von Materialien, etc.

Hierbei werden geladene Partikel wie z.B. Protonen oder Kohlenstoffionen oder andere Ionen auf hohe Energien beschleunigt, zu einem Partikelstrahl geformt und über ein Hochenergiestrahltransportsystem zu einem oder mehreren Bestrahlungsräumen geführt. In dem Bestrahlungsraum wird das zu bestrahlende Zielvolumen mit dem Partikelstrahl bestrahlt.

Es kann dabei vorkommen, dass sich das zu bestrahlende Zielvolumen bewegt. Beispielsweise kann bei der Bestrahlung eines Patienten eine Bewegung des zu bestrahlenden Tumors durch eine Atembewegung verursacht werden. Eine derartige Bewegung kann auch anhand von als Phantomen bezeichneten Modellobjekten für Forschungszwecke nachgebildet werden.

Insbesondere bei Bestrahlungsverfahren, bei denen eine Vielzahl von Bestrahlungsdosen sukzessive an unterschiedlichen Orten im Zielvolumen deponiert werden soll, also bei einem gescannten Partikelstrahl, ist es schwer, eine gewünschte homogene Dosisverteilung im Zielvolumen zu erreichen, wenn sich das Zielvolumen während des Fortgangs der Bestrahlung bewegt.

Eine bekannte Möglichkeit, die Bewegung des Zielvolumens zu kompensieren, sind Bestrahlungsverfahren, die unter den Begriffen "Rescanning", "Gating" und "Tracking" bekannt sind. Unter "Rescanning" wird verstanden, dass der Strahl mehrfach wie geplant appliziert wird, so dass sich Fehldosierungen der einzelnen Durchläufe ausmitteln. Unter "Gating" wird verstanden, dass der Strahl nur zu festgelegten Fenstern der Bewegung appliziert wird, so dass der Bewegungseinfluss gemindert oder bestenfalls sogar ausgeschlossen wird. Unter "Tracking" wird verstanden, dass der Strahl, mit dem die Bestrahlung des Zielvolumens erfolgt, der Bewegung des Zielvolumens nachgeführt wird. Falls der Strahl ein Partikelstrahl ist, kann dies beispielsweise erreicht werden, indem ein Strahl durch Magnetsysteme derart abgelenkt wird, dass sein Verlauf der Bewegung des Zielvolumens nachgeführt wird. Gegebenenfalls kann der Strahl auch in seiner Energie variiert werden, um die Eindringtiefe des Strahls der Bewegung des Zielvolumens anzupassen. Auch bei einer Bestrahlung mit Photonen kann ein Tracking durchgeführt werden. Dies kann z.B. durch eine Veränderung des den Strahl begrenzenden Kollimators erfolgen, wobei die Kollimatoröffnung an die Bewegung des Zielvolumens angepasst wird.

Aus der US 6,891,177 B1, der US 6,710,362 B2 und der US 2006/0033042 A1 sind Verfahren und Vorrichtungen bekannt, mit denen eine Bewegungsnachführung des Strahls durchgeführt werden kann.

Aus der DE 10 2007 014 715 A1 sind Verfahren und Vorrichtungen bekannt, mit denen Steuerparametern für eine Bestrahlung eines bewegten Zielvolumens in einem Körper bestimmt werden, wobei die im bestrahlten Körper erzeugten Dosisverteilung mittels einer PET-Kamera ermittelt wird.

Es ist die Aufgabe der Erfindung, ein Verfahren und eine Bestrahlungsanlage bereitzustellen, welche auch bei Bestrahlung eines Zielvolumens unter Bewegungsnachführung des Strahls eine genaue Dosisdeposition ermöglichen.

Die Aufgabe wird durch die unabhängigen Ansprüche gelöst. Bevorzugte Ausgestaltungen der Erfindung sind in den abhängigen Ansprüchen angegeben und werden im Folgenden näher erläutert. Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Das erfindungsgemäße Verfahren zur Bestrahlung eines sich bewegenden Zielvolumens wird in dem Anspruch 1 definiert.

Bei einer Bestrahlung, bei der der Strahl der Bewegung des Zielvolumens nachgeführt wird, d.h. bei sogenannten Tracking-Verfahren, führt das Tracking zu einer korrekten Deposition der Zieldosis im Zielvolumen. Interferenzeffekte, die dadurch entstehen, dass sich das Zielvolumen während der Bestrahlung bewegt, und die bewirken, dass es bei nicht nachgeführtem Strahl an Stellen im Zielvolumen zu einer Über- bzw. Unterdosierung in Bezug auf die geplante Dosisverteilung kommen kann, werden auf diese Weise weitgehend vermieden.

Das erfindungsgemäße Verfahren beruht auf der Erkenntnis, dass gerade das Tracking, das zur Vermeidung derartiger Interferenzeffekte im Zielvolumen eingesetzt wird, allerdings zu ähnlichen "inversen" Interferenzeffekten führen kann, und zwar in Bereichen, die bei der Bestrahlung des Zielvolumens ebenso mit einer Dosis belastet werden, wie beispielsweise Bereiche im Eintrittskanal des Strahls. Dies tritt dann auf, wenn der Bereich ein anderes Bewegungsmuster als das Zielvolumen aufweist, da der Strahl an die Bewegung des Zielvolumens angepasst nachgeführt wird und nicht an die andersartigen Bewegung des Bereichs.

Doch selbst wenn sich der Bereich gleichartig wie das Zielvolumen bewegt, kann ein derartiger "inverser" Interferenzeffekt auftreten. Dies geschieht nämlich auch dann, wenn sich durch die Bewegung des Zielvolumens eine Partikelreichweitenänderung bezüglich der Geometrie des Zielvolumens ergibt, die für das Zielvolumen anders ist als für den Bereich. Beispielsweise kann im Eintrittskanal vor dem Zielvolumens eine stationäre Struktur (z.B. ein Knochen) liegen, die die Reichweite des Partikelstrahls beeinflusst. Wenn sich das dahinterliegende Zielvolumen bewegt und mit dem Partikelstrahl "getrackt" werden soll, muss die Bewegungsnachführung die sich verändernde Reichweite des Partikelstrahls, die sich aus der Relativbewegung Knochen - Zielvolumen ergibt, berücksichtigen. Wenn nun aber vor dem Knochen ein weiterer Bereich liegt, der sich gleich wie das Zielvolumen bewegt, kann die Bewegungsnachführung zu Interferenzeffekten in diesem Bereich führen. Dies rührt letztlich daraus, dass die Partikelreichweitenänderung das Zielvolumen betrifft, nicht aber den vor dem Knochen liegenden Bereich.

Der Bereich kann z.B. ein weiteres Volumen sein, das ebenso wie das sich bewegende Zielvolumen in dem zu bestrahlenden Objekt liegt, wie beispielsweise ein zu schonendes Risikoorgan, z.B. die Haut im Eintrittskanal eines Strahls. Der Bereich kann auch ein Bereich sein, der gemäß einer ursprünglichen Bestrahlungsplanung überhaupt nicht mit einer Dosis belastet wird. Eine Dosisbelegung, die sich aus Interferenz des nachgeführten Strahls mit einem derartigen Bereich ergibt, ist in einer herkömmlichen Referenzbestrahlungsplänen nicht hinterlegt bzw. kann aus Referenzbestrahlungsplänen nicht ohne weiteres entnommen werden.

Der Bereich kann neben den oben geschilderten Volumina aber auch beispielsweise der Bereich einer Detektionsvorrichtung sein, die vor oder hinter dem Objekt angeordnet ist, in welchem das Zielvolumen ist.

Insbesondere wenn das Zielvolumen mit gescannten Partikelstrahlen bestrahlt wird bzw. bestrahlt werden soll, treten Interferenzeffekte deutlich auf aufgrund der genauen und gezielten Dosisdeposition bei einem Partikelstrahl.

Es kann daher der Fall eintreten, dass der Bereich mit einer Dosis belegt bzw. belastet wird, die zuvor nicht geplant war und die nicht ohne weiteres vorhersehbar war. Die tatsächliche Dosis, mit der der Bereich belegt bzw. belastet wird, hängt von der tatsächlich durchgeführten Bewegungsnachführung ab. Die Dosisbelegung kann somit unter Verwendung der tatsächlich während der Bestrahlung durchgeführten Bewegungsnachführung ermittelt werden.

In einer vorteilhaften Variante des Verfahrens wird beim Ermitteln der Dosisbelegung eine Dosisberechnung durchgeführt, mit der eine Dosis bestimmt wird, die in dem Bereich deponiert wird, wobei die Dosisberechnung unter Verwendung der tatsächlich während der Bestrahlung durchgeführten Bewegungsnachführung erfolgt.

Die Dosisberechnung kann dabei auf verschiedene Weise durchgeführt werden. Es kann z.B. eine Berechnung der Dosisverteilung für den Bereich durchgeführt werden, die Berechnung eines Dosis-Volumen-Histogramms, die Berechnung der maximalen Dosis, oder ähnliche bekannte Verfahren aus der Strahlentherapie, die eingesetzt werden, um eine Dosisbelastung für einen Bereich anzugeben.

Welche Dosisbelegung genau in dem Bereich auftritt, hängt dabei von der tatsächlichen Bewegung des Zielvolumens während der Bestrahlung im Tracking-Verfahren ab. Zur Ermittlung der Dosisbelegung in dem Bereich, insbesondere bei einer Dosisberechnung, können deshalb Daten verwendet werden, welche die Bewegungsnachführung des Strahls während der Bestrahlung des Zielvolumens kennzeichnen. Hierzu können z.B. die Werte eines Therapie-Online-Monitors, eines Sensor zur Detektion der Bewegung des Zielvolumens und der weiteren Volumina lateral, proximal oder distal des Zielvolumens, die Trajektorie eines Bewegungserfassungssystems, die extrahierte Intensität, usw. protokolliert werden. Die Detektion der Bewegung von Volumina kann anhand der detektierten Bewegungsphase über ein Surrogat erfolgen, wenn der Bewegungsmonitor nicht direkt die 3D Information liefert.

Beispielsweise kann aus den aufgezeichneten Werten bzw. Daten die konkrete Lage des Strahls im zu bestrahlenden Objekt ermittelt werden, d.h. Informationen darüber, wie sich der Strahl im zu bestrahlenden Objekt bewegt hat oder wann und wo sich der Strahl bei der Bestrahlung im Objekt befunden hat.

In einer vorteilhaften Ausführungsform wird als Dosisbelegung ein Bestrahlungsmuster ermittelt, das in dem Bereich durch die Bestrahlung hervorgerufen wird. Ein Bestrahlungsmuster kann oftmals mit weniger Aufwand ermittelt werden, als es für eine konkrete Dosisberechnung notwendig ist.

Beispielsweise kann die Dosisbelegung bzw. das Bestrahlungsmuster mithilfe einer Strahlungsdetektionsvorrichtung ermittelt und/oder gemessen werden.

Als Strahlungsdetektionsvorrichtung können verschiedene bekannte Vorrichtungen verwendet werden, beispielsweise ein radiographischer Film, ein zweidimensionaler Szintillationsdetektor, oder ein anderer geeigneter Flächendetektor. Der Bereich, für den die Dosisbelegung ermittelt wird, kann z.B. der mit der Strahlung wechselwirkende Teil der Detektionsvorrichtung sein. Alternativ kann beispielsweise die Kombination aus Bewegungsmonitor, Vieldrahtkammer zur Messung der Strahllage und Ionisationskammer zur Messung der eingestrahlten Strahlintensität verwendet werden.

Das Bestrahlungsmuster, das durch die Detektionsvorrichtung ermittelt bzw. gemessen wird, kann z.B. als Maß für die Dosisbelegung des Bereichs verwendet werden, wenn die Detektionsvorrichtung und der Bereich eine ähnliche Bewegung aufweisen. Wenn im Bestrahlungsmuster der Detektionsvorrichtung deutliche Inhomogenitäten auftreten, kann dies als Zeichnen gewertet werden, dass in dem Bereich ähnliche Inhomogenitäten aufgetreten sind.

Das Bestrahlungsmuster, das mithilfe der Detektionsvorrichtung ermittelt wird, erlaubt es auch, das tatsächlich durchgeführte Tracking zu messen und zu dokumentieren, auch wenn die das Tracking kennzeichnenden Daten nicht notwendigerweise mitprotokolliert wurden.

Erfindungsgemäß wird die Dosisbelegung zur Überprüfung der Bewegungsnachführung verwendet.

Insbesondere kann das Bestrahlungsmuster, das mithilfe der Detektionsvorrichtung ermittelt wird, zur Überprüfung der Bewegungsnachführung verwendet werden. Beispielsweise kann die Bewegungsnachführung dadurch überprüft werden, indem die ermittelte Dosisbelegung mit einer zu erwartenden Dosisbelegung verglichen wird.

Das mit der Detektionsvorrichtung ermittelte Bestrahlungsmuster gibt z.B. Aufschluss darüber, wie der Strahl bei der Bewegungsnachführung tatsächlich nachgeführt wurde. Aus den Daten, die das Tracking des Strahls kennzeichnen und die während der Bestrahlung z.B. protokolliert worden sind, kann aber auch das Bestrahlungsmuster ermittelt werden, das mit der Detektionsvorrichtung hätte ermittelt werden sollen. Es kann hierfür z.B. das Sensorsignal verwendet werden, mit dem die Bewegung des Zielvolumens während der Bestrahlung überwacht wird und aufgrund dessen die Bewegungsnachführung des Strahls gesteuert wird.

Ein Vergleich des "Soll-Bestrahlungsmusters" mit dem tatsächlich ermittelten "Ist-Bestrahlungsmusters" erlaubt dann einen Rückschluss, ob das Tracking wie gewünscht durchgeführt wurde, und kann somit zur Qualitätskontrolle einer Bestrahlung eingesetzt werden. Ein Anwender ist dadurch gewarnt und kann beispielsweise erneut qualitätssichernde Maßnahmen, wie eine Überprüfung des Tracking-Systems, durchführen, oder eine Wiederholung der Bildgebung und/oder eine Anpassung der Bestrahlungsplanung.

In einer vorteilhaften Ausführungsform kann der Vergleich auch während der Bestrahlung durchgeführt werden. Im Falle von Abweichungen wird die Bestrahlung unterbrochen oder abgebrochen (Interlock). Üblicherweise werden lediglich Abweichungen, welche über einem Schwellwert liegen, zu einem Abbruch führen.

In einer vorteilhaften Ausführungsform wird eine nachfolgende Bestrahlung des Zielvolumens anhand der ermittelten Dosisbelegung modifiziert.

Dies kann dadurch erfolgen, dass insbesondere bei einer fraktionierten Bestrahlung die Bestrahlungsplanung modifiziert oder neu durchgeführt und an die Dosis angepasst wird, mit der der Bereich bereits belastet worden ist. Es kann aber auch ein Bestrahlungsverfahren, d.h. eine Bestrahlungsart oder Bestrahlungstechnik, geändert werden. Wenn es beispielsweise zu großen Inhomogenitäten in dem Bereich gekommen ist, kann z.B. festgelegt werden, für die nachfolgenden Bestrahlungen ein Gating-Verfahren zu verwenden anstelle eines Tracking-Verfahrens.

Unter Verfahren zur Bestrahlung werden hier sowohl Verfahren verstanden, bei denen tatsächlich eine Bestrahlung eines Zielvolumens durchgeführt wird, als auch Verfahren, bei denen eine Bestrahlung eines Zielvolumens lediglich simuliert wird. Bei einer simulierten Bestrahlung wird der Strahl bei denen der Strahl einer "virtuellen", also simulierten, Bewegung des Zielvolumens nachgeführt.

Derartige Simulationen werden oftmals im Rahmen einer Bestrahlungsplanung eingesetzt und liefern Informationen, ob eine Bestrahlung den gewünschten Kriterien entspricht oder ob eine Bestrahlung geändert werden soll. Beispielsweise können Bestrahlungen für eine Vielzahl von unterschiedlichen Bewegungsmustern des Zielvolumens simuliert werden. Bei jeder dieser simulierten Bestrahlungen kann die Dosisbelegung ermittelt werden, die in dem vom Zielvolumen unterschiedlichen Bereich deponiert würde und die sich aufgrund des Bewegungsmusters des Zielvolumens und der sich damit zusammenhängenden Bewegungsnachführung ergeben würde.

Wird diese Simulation bei einer Vielzahl von unterschiedlichen Bewegungsmustern des Zielvolumens durchgeführt, ohne dass unerwünschte oder gar gefährliche Überdosierungen in dem vom Zielvolumen unterschiedlichen Bereich auftreten, kann angenommen werden, dass auch bei einer tatsächlich durchgeführten Bestrahlung keine Überdosierungen auftreten. Auf diese

Weise kann beispielsweise simuliert werden, ob es durch die Bewegungsnachführung zu einer gefährlichen Überdosierung in einer Risikostruktur kommen könnte.

Auch für den Fall, dass die Dosisbelegung in dem Bereich für eine Vielzahl von Bewegungsmustern des Zielvolumens simuliert worden ist, kann eine Überprüfung der tatsächlich applizierten Dosisverteilung in dem Bereich trotzdem sinnvoll sein, falls tatsächlich eine Bestrahlung durchgeführt wird.

Das Verfahren kann also einerseits bei therapeutischen Behandlungen eingesetzt werden, bei denen eine tatsächliche Bestrahlung eines menschlichen oder tierischen Körpers stattfindet. Das Verfahren kann aber andererseits und erfindungsgemäß als nichttherapeutisches Verfahren eingesetzt werden, beispielsweise indem eine Bestrahlung lediglich simuliert wird oder in dem eine Bestrahlung von anderen Gegenständen als einen menschlichen oder tierischen Körper durchgeführt wird, wie z.B. die Bestrahlung eines Phantoms oder die Bestrahlung von Materialien im Allgemeinen.

Die erfindungsgemäße Bestrahlungsanlage zum Bestrahlen eines sich bewegenden Zielvolumens wird in dem Anspruch 7 definiert.

Die Bestrahlungsanlage, insbesondere die Bestrahlungsvorrichtung, kann dabei ausgebildet sein zum Durchführen einer tatsächlichen Bestrahlung und/oder zum Durchführen einer lediglich simulierten Bestrahlung.

Die Überwachungsvorrichtung kann eine Strahlungsdetektionsvorrichtung umfassen, mit der die Dosisbelegung, die während einer Bestrahlung in dem Bereich auftritt, ermittelt bzw. gemessen werden kann.

Die Bestrahlungsanlage kann eine Bestrahlungsteuerungsvorrichtung umfassen zum Modifizieren einer nachfolgenden Bestrahlung des Zielvolumens oder zum Ändern einer Bestrahlungsplanung für das Zielvolumen unter Verwendung der ermittelten Dosisbelegung. Weiterhin kann die Bestrahlungsanlage eine Erfassungseinrichtung zum Aufzeichnen von Daten umfassen, aus welchen sich eine während der Bestrahlung durchgeführte Bewegungsnachführung ermitteln bzw. rekonstruieren lässt.

Ausführungsformen der Erfindung sowie vorteilhafte Weiterbildungen gemäß den Merkmalen der abhängigen Ansprüche werden anhand der folgenden Zeichnung näher erläutert, ohne jedoch darauf beschränkt zu sein. Hierbei sind in allen Figuren gleiche Gegenstände mit gleichen Bezugszeichen versehen.
Fig. 1 zeigt eine Bestrahlungsanlage gemäß einer Ausführungsform der Erfindung;
Fig. 2 zeigt eine Darstellung eines Ausschnittes der Bestrahlungsanlage, in der eine Ausführung des erfindungsgemäßen Verfahrens ausgeführt werden kann;
Fig. 3 und Fig. 4 zeigen eine Darstellung eines Ausschnittes der Bestrahlungsanlage, in der weitere Ausführungen des erfindungsgemäßen Verfahrens ausgeführt werden können;
Fig. 5 zeigt eine Dosisbelegung für ein bestrahlten, bewegten Zielvolumens und einen bestrahlten ruhenden Bereichs zur Illustration von Interferenzeffekten;
Fig. 6 zeigt ein schematisches Ablaufdiagramm einer Ausführungsform des erfindungsgemäßen Verfahrens.

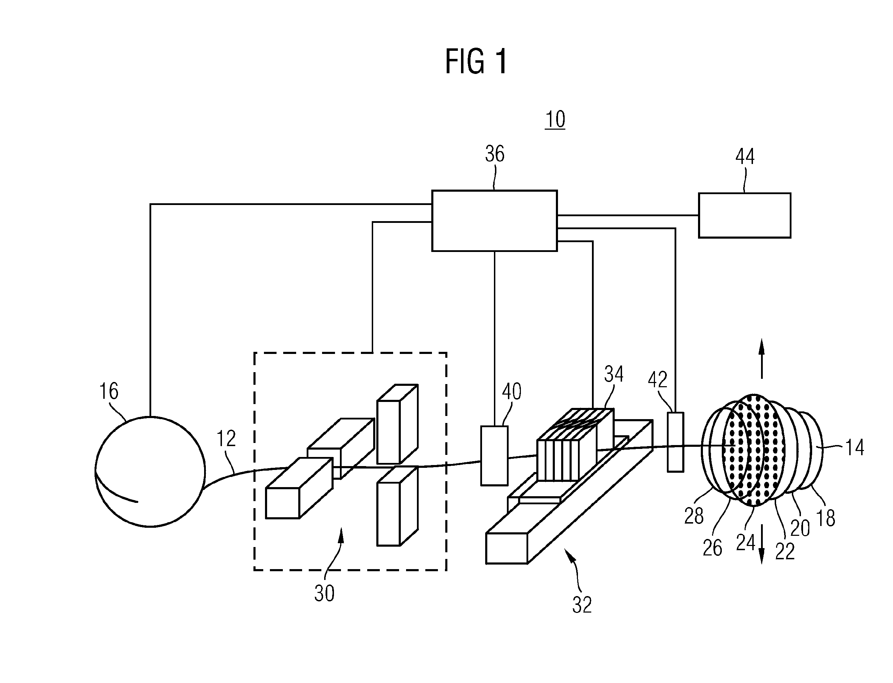

Figur 1 zeigt in stark schematisierter Darstellung einen Aufbau einer als Partikeltherapieanlage 10 aufgebauten Bestrahlungsanlage. Die Partikeltherapieanlage 10 wird zur Bestrahlung eines auf einer Positioniervorrichtung angeordneten Körpers mit einem Strahl aus Partikeln, der im Folgenden als Partikelstrahl 12 bezeichnet ist, verwendet. Insbesondere kann ein tumorerkranktes Gewebe 14 eines Patienten mit dem Partikelstrahl 12 bestrahlt werden. Es ist ebenfalls vorgesehen, die Partikelstrahlanlage 10 zur Bestrahlung eines nicht-lebenden Körpers, insbesondere eines Wasserphantoms oder anderen Phantomen einzusetzen. Die Bestrahlung des Wasserphantoms kann beispielsweise zu Zwecken der Überprüfung und Verifizierung von Bestrahlungsparametern vor und/oder nach einer erfolgten Bestrahlung eines Patienten erfolgen. Es ist aber auch vorgesehen, andere Körper, insbesondere Versuchsaufbauten wie beispielsweise Zellkulturen oder Bakterienkulturen zu Forschungszwecken mit dem Partikelstrahl 12 zu bestrahlen. In allen Fällen kann es sich um bewegte oder ruhende Körper handeln.

Die Partikeltherapieanlage 10 weist typischerweise eine Beschleunigereinheit 16 auf, z.B. ein Synchrotron, ein Zyklotron oder einen sonstigen Beschleuniger, der einen Partikelstrahl 12 mit der zur Bestrahlung notwendigen Energie bereitstellt. Als Partikel werden vornehmlich Teilchen wie beispielsweise Protonen, Pionen, Heliumionen, Kohlenstoffionen oder Ionen anderer Elemente eingesetzt. Typischerweise hat einen Partikelstrahl 12 einen Strahldurchmesser von 3-10 mm.

Die Bestrahlungsanlage kann aber auch eine Elektronenstrahlvorrichtung sein, die einen Elektronenstrahl zur Bestrahlung eines Zielvolumens 14 bereitstellt oder eine Röntgenbestrahlungseinrichtung. Insbesondere in letzterem Fall kann ein Tracking auch mit einer sich bewegenden Kollimatoröffnung durchgeführt werden.

In dem zu bestrahlenden Zielvolumen 14 sind Schichten 18, 20, 22, 24, 26 und 28 angedeutet, welche isoenergetischen Schichten entsprechen. Eine isoenergetische Schicht 18, 20, 22, 24, 26 oder 28 entspricht jeweils Eindringtiefe des Braggpeaks für eine bestimmte Energie des Partikelstrahls 12.

Als Scanverfahren wird bevorzugt ein Rasterscan-Verfahren verwendet, bei dem ein Partikelstrahl von Rasterpunkt zu Rasterpunkt geführt wird ohne zwangsläufige Abschaltung bei einem Übergang von einem Rasterpunkt zum nächsten. Es können auch Spotscan-Verfahren mit Abschaltung des Partikelstrahls zwischen den einzelnen Rasterpunkten oder andere Scan-Verfahren wie beispielsweise kontinuierliche Scanverfahren eingesetzt werden. In Fig. 1 ist das Scan-Verfahren schematisch illustriert, anhand einiger schwarzer Punkte, welche in einem schichtweise aufgebauten Zielvolumen 14 dargestellt sind und welche sukzessive mit dem Partikelstrahl angefahren werden.

Der Partikelstrahl 12 wird in seiner lateralen Auslenkung mithilfe von Scan-Magneten 30 beeinflusst, d.h. in seiner Position senkrecht zu Strahlverlaufsrichtung, auch als x- und y-Richtung bezeichnet, abgelenkt. Eine longitudinale Beeinflussung, also eine Variation der Eindringtiefe des Partikelstrahls entlang der Strahlrichtung, wird bevorzugt mittels einer passiven Energievariationseinrichtung 32 durchgeführt, die beispielsweise bewegliche Keile 34 aufweisen kann, welche über einen linearen Motor (nicht dargestellt) bewegt werden. Mithilfe der Keile kann die Dicke des Materials eingestellt werden, durch das der Partikelstrahl gelenkt wird, und damit eine Energiemodulation des Partikelstrahls 12 erreicht werden. Die Energievariationseinrichtung kann auch proximal der Scannermagnete positioniert sein und kann auch beispielsweise in Form eines binären Plattensystems konstruiert sein oder gar über ein spezielles Synchrotron gelöst werden oder auch direkt vom Energieselektionssystem eines Zyklotrons gelöst werden.

Die Bestrahlungsanlage 10 weist ferner eine Ablaufsteuerung 36 auf, Detektoren zur Überwachung der Strahlparameter 40, sowie fakultativ eine Strahlungsdetektionsvorrichtung 42, welche beispielsweise unmittelbar vor dem Zielvolumen 14 positioniert sein kann. Die Anordnung der hier gezeigten Komponenten der Partikelstrahlanlage ist lediglich beispielhaft. Auch Anordnungen in anderer Reihenfolge sind denkbar.

Die Ablaufsteuerung 36, also das Kontrollsystem der Anlage, steuert die einzelnen Komponenten der Anlage, wie beispielsweise den Beschleuniger 16, die Scan-Magnete 30 und das Energievariationseinrichtung 32, und sammelt Messdaten wie beispielsweise die Daten der Detektoren zur Überwachung der Strahlparameter 40. Üblicherweise erfolgt die Steuerung aufgrund eines Bestrahlungsplans, der von einer Bestrahlungsplanungseinrichtung 44 bereitgestellt wird.

Der hier gezeigte Aufbau ist beispielhaft für einen möglichen Aufbau einer Partikeltherapieanlage 10. In einer derartigen Partikeltherapieanlage können Ausführungsformen der Erfindung implementiert werden. Mögliche Ausführungsformen werden anhand der nachfolgenden Zeichnung näher erläutert.

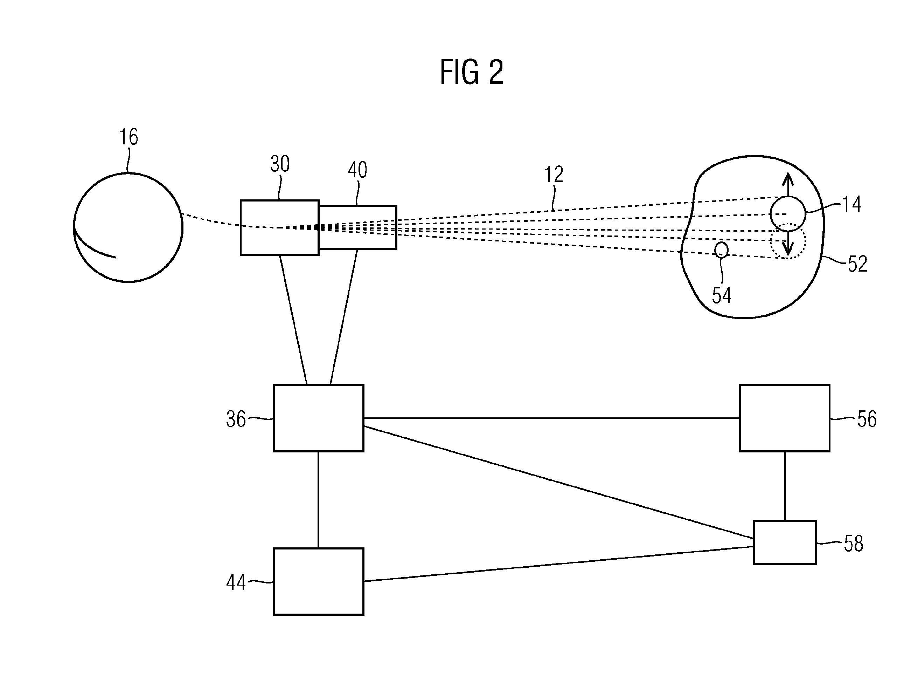

Fig. 2 zeigt in schematischer Darstellung ein zu bestrahlendes Zielobjekt 52, in dem sich das zu bestrahlende Zielvolumen 14 befindet. Ferner ist ein weiterer Bereich 54 gezeigt, der sich in dem zu bestrahlenden Zielobjekt 52 befindet, und er beispielsweise ein zu schonendes Risikoorganen darstellen kann. Durch die Bestrahlung des Zielvolumens 14 wird auch der weitere Bereich 54 mit einer Dosis belastet. Das Zielvolumen 14 bewegt sich dabei während der Bestrahlung, angedeutet durch die Pfeile. Der weitere Bereich 54 bewegt sich nicht und weist damit ein anderes Bewegungsmuster auf als das Zielvolumen 14.

Wenn der Partikelstrahl 12 der Bewegung des Zielvolumens 30 bei der Bestrahlung nachgeführt wird, wird dadurch erreicht, dass das Zielvolumen 14 mit einer gewünschten Dosisverteilung belegt wird. Um dies zu erreichen, ist eine Bewegungsdetektionsvorrichtung 56 vorgesehen, z.B. ein externer Sensor oder ein Fluoroskopiesystem, mit der die während einer Bestrahlung auftretende Bewegung des Zielvolumens 14 detektiert werden kann. Mithilfe der so erzeugten Messdaten kann die Ablaufsteuerung 36 den Partikelstrahl 12 entsprechend steuern, so dass der Partikelstrahl 12 der Bewegung des Zielvolumens 14 nachgeführt wird.

Abhängig von der tatsächlich durchgeführten Bewegung des Zielvolumens 14 kann es jedoch vorkommen, dass es in dem weiteren Bereich 54 zu unvorhergesehenen Überlagerungseffekten kommt, wodurch der Bereich 54 mit einer Dosis beziehungsweise Dosisverteilung belegt werden kann, die von einer ursprünglichen Planung abweicht und/oder die nicht mehr tolerierbar ist.

Als Überwachungsvorrichtung 58, mit der diese Dosisbelegung detektiert werden kann, ist eine Rechnereinheit vorgesehen. Diese Rechnereinheit bekommt als Eingangsdaten die Messdaten der Bewegungsdetektionsvorrichtung 56 und/oder Daten, welche die Nachführung des Partikelstrahls 12 beschreiben, z.B. Steuerdaten, die zur Steuerung der Scan-Magnete 30 und der Energievariationseinrichtung verwendet wurden. Aus diesen Daten kann die Überwachungsvorrichtung 58 die Dosisbelegung ermitteln, mit der der sich nicht bewegende Bereich 54 bei der Bestrahlung tatsächlich belegt worden ist.

Diese ermittelte Dosisbelegung kann dann wiederum der Ablaufsteuerung 36 zur Verfügung gestellt werden, welche hierauf basierend den Bestrahlungsverlauf modifizieren kann, oder aber auch einer Bestrahlungsplanungsvorrichtung 44, welche einen bestehenden Bestrahlungsplan für nachfolgende Sitzungen modifizieren kann.

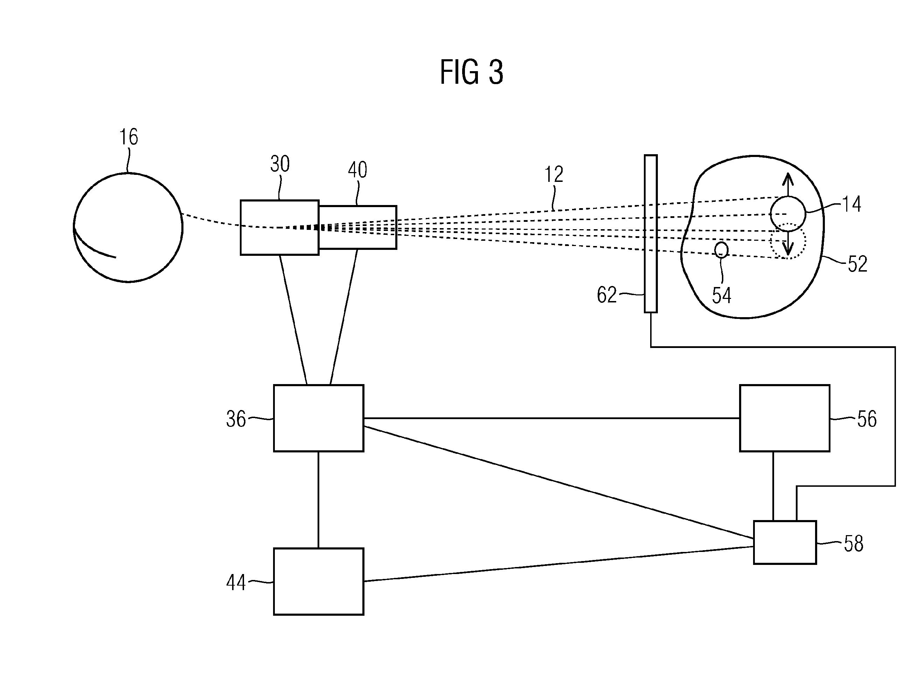

Fig. 3 unterscheidet sich von Fig. 2 insofern, als dass ein Detektor 62 als Strahlungsdetektionsvorrichtung 42 zum Aufzeichnen eines Bestrahlungsmusters vor Zielobjekt 52 angeordnet ist. Der Detektor 62 ist dabei ruhend, d.h. er weist ein anderes Bewegungsmuster als das Zielvolumen 14 auf.

Der Partikelstrahl bestrahlt den strahlungssensitiven Bereich im Detektor 42, erzeugt folglich im Detektor 42 eine Dosisbelegung. Das mit dem Detektor 62 aufgezeichnete Bestrahlungsmuster kann z.B. dazu verwendet werden, um die Dosisbelegung, mit der der Bereich 54 im Zielvolumen 14 belegt worden ist, zu ermitteln. Hierzu kann beispielsweise das aufgezeichnete Bestrahlungsmuster der Überwachungsvorrichtung 58 übermittelt werden, die unter Verwendung dieser Daten die Dosisbelegung des Bereichs 54 ermittelt. Das Bestrahlungsmuster kann deshalb zur Berechnung der Dosisbelegung verwendet werden, da das Bestrahlungsmuster die laterale Position des Partikelstrahls 12, die sich aufgrund der Bewegungsnachführung ergibt, widerspiegelt, welche wiederum mit der Dosisbelegung des Bereichs 54 korreliert ist.

Das mit dem Detektor 62 aufgezeichnete Bestrahlungsmuster - also die durch den Detektor 62 direkt ermittelte Dosisbelegung des Detektors 62 - kann aber auch direkt dazu verwendet werden, die tatsächlich durchgeführte Bewegungsnachführung zu verifizieren. Aus den Steuerdaten, die zur Durchführung der Bewegungsnachführung eingesetzt werden, kann das "Soll-Bestrahlungsmuster" ermittelt werden, das sich bei korrekt durchgeführter Bewegungsnachführung ergeben soll. Ein Vergleich mit dem aufgezeichneten "Ist-Bestrahlungsmuster" kann dann aufgetretene Ungenauigkeiten bei der Bewegungsnachführung aufdecken. Diese Verifikation kann evtl. sogar während der Bestrahlung durchgeführt werden, und dann ggf. einen Interlock setzen, d.h. die Bestrahlung bei Auffälligkeiten abbrechen.

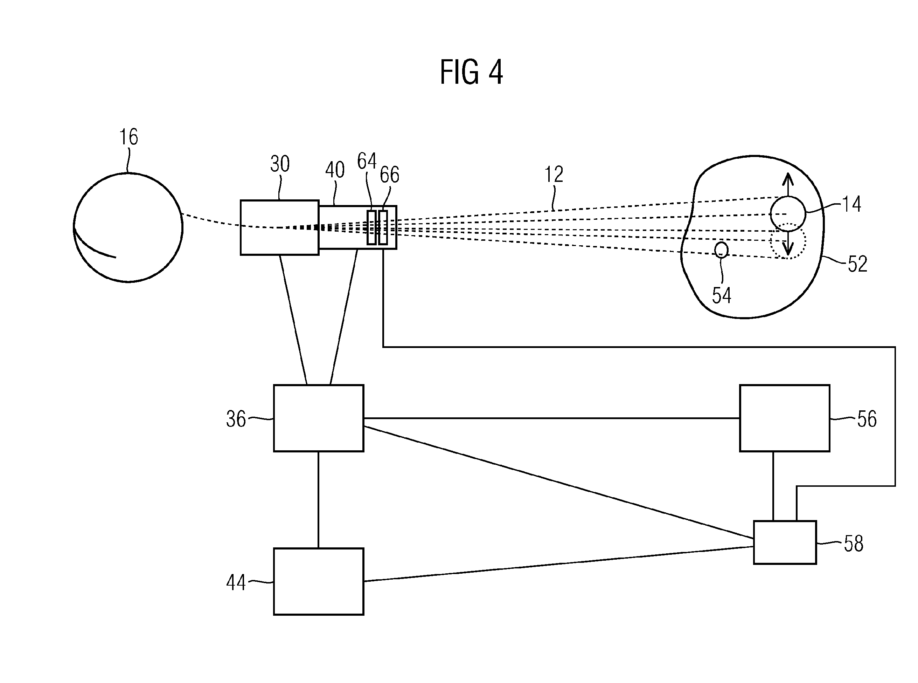

Fig. 4 zeigt im Gegensatz zu Fig. 3, dass anstelle des vor dem Zielobjekt 52 positionierten Detektors 62 auch die Detektoren zur Überwachung der Strahlparameter 40 verwendet werden. Derartige Detektoren sind oftmals in der in der Strahlapplikationseinheit, auch Nozzle genannt, angeordnet. Diese Direktoren umfassen üblicherweise einen Ortsdetektor 64 (z.B. einen MWPC für engl.: "multi-wire proportional chamber") und einen Intensitätsdetektor 66 (z.B. eine Ionisationskammer). Eine Kombination der Messdaten des Ortsdetektors 64 und des Intensitätsdetektors 66 erlaubt es, das Bestrahlungsmuster, das durch den Partikelstrahl verursacht wurde, zu ermitteln. Vorteil dieser Ausführungsvariante ist, dass keine zusätzlichen Detektoren bereitgestellt werden müssen, sondern bereits vorhandene Detektoren verwendet werden können.

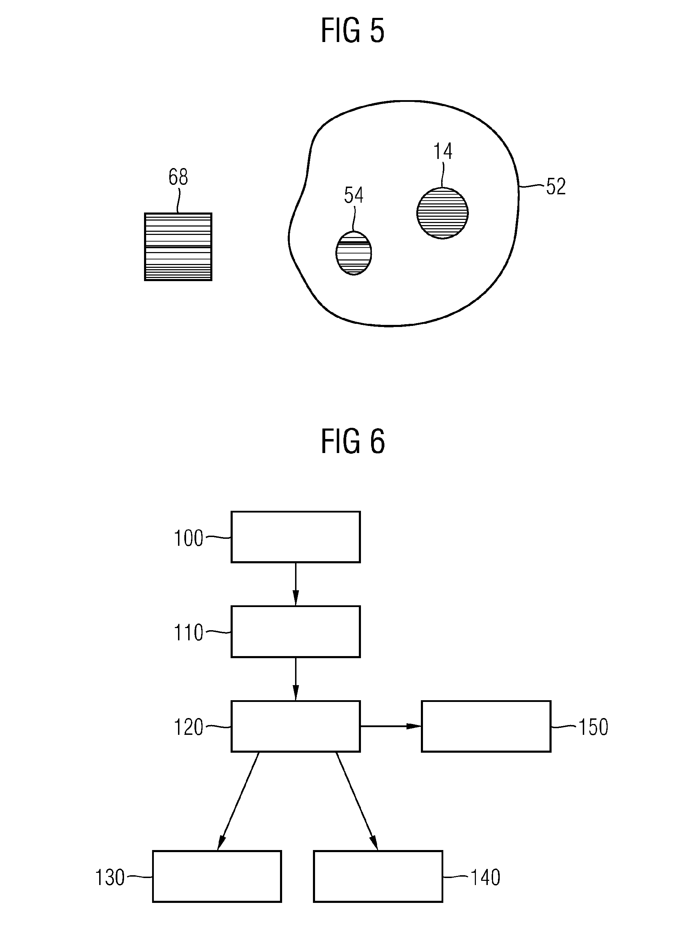

Fig. 5 zeigt in vergrößerter Ansicht nochmals das Zielobjekt 52 mit dem Zielvolumen 14 und dem vor dem Zielvolumen 14 liegenden Bereich 54. Aufgrund der Strahlnachführung des Partikelstrahls während der Bestrahlung ist in dem Zielvolumen 14 eine gleichmäßige Dosisverteilung appliziert worden, angedeutet durch die gleichmäßige Schraffur des Zielvolumens 14. Da sich während der Bestrahlung der Bereich 54 nicht oder anders bewegt hat, führt dies zu einer ungleichmäßigen Dosisbelegung in dem Bereich 54, symbolisiert durch die ungleichmäßige Schraffur. Der ungleichmäßigen Dosisbelegung entspricht ein Bestrahlungsmuster 68, das mit einem vor dem Zielobjekt 52 positionierten Detektor 62 aufgezeichnet wurde.

Wie bereits erläutert, tritt dieser "Interferenzeffekt" im vor dem Zielvolumen 14 liegenden Bereich 54 auch dann auf, wenn sich Zielvolumen 14 und Bereich 54 gleichartig bewegen, wenn aber aufgrund von zwischen Bereich 54 und Zielvolumen liegenden Strukturen eine Reichweitenänderung des Partikelstrahls ergibt, die für das Zielvolumen 14 anders ist als für den Bereich 54.

Fig. 6 zeigt schematisch Verfahrensschritte, die in einer möglichen Ausführungsform des Verfahrens durchgeführt werden können.

In einem ersten Schritt (Schritt 100) wird das bewegte Zielvolumen unter Bewegungsnachführung bestrahlt. Während der Bestrahlung wird die tatsächlich während der Bestrahlung eingesetzte Bewegungsnachführung ermittelt (Schritt 110). Anhand der tatsächlich eingesetzten Bewegungsnachführung wird eine Dosisbelegung ermittelt (Schritt 120), welche in einem Bereich aufgetreten ist, der ein unterschiedliches Bewegungsmuster als das Zielvolumen aufweist. Kann die ermittelte Dosisbelegung toleriert werden, so kann die Bestrahlung wie geplant weiter fortgeführt werden (Schritt 130).

Andernfalls kann die Bestrahlung des Zielvolumens oder eine Bestrahlungsplanung für das Zielvolumen geändert werden (Schritt 140). Alternativ kann die Dosisbelegung verwendet werden, um die tatsächlich durchgeführte Bewegungsnachführung zu verifizieren, z.B. durch einen Vergleich eines Soll-Bestrahlungsmusters mit einem Ist-Bestrahlungsmuster (Schritt 150). Ein Abbruch der Bestrahlung (Interlock) kann ggf. gesetzt werden.

Alle im Zusammenhang mit den Figuren gezeigten Ausführungsformen, welche anhand einer tatsächlichen Bestrahlung erklärt und beschrieben wurden, können ebenso lediglich simuliert werden, also im Rahmen einer simulierten Bestrahlung angewendet werden.

### Bezugszeichenliste

- 10: Partikeltherapieanlage
- 12: Partikelstrahl
- 14: Zielvolumen
- 16: Beschleunigereinheit
- 18 - 28: Schichten
- 30: Scan-Magnete
- 32: Energievariationseinrichtung
- 34: Keile
- 36: Ablaufsteuerung
- 40: Detektoren zur Überwachung der Strahlparameter
- 42: Strahlungsdetektionsvorrichtung
- 44: Bestrahlungsplanungsseinrichtung

- 52: Zielobjekt
- 54: Bereich
- 56: Bewegungsdetektionsvorrichtung
- 58: Überwachungsvorrichtung
- 62: Detektor
- 64: Ortsdetektor
- 66: Intensitätsdetektor
- 68: Bestrahlungsmuster

- 100: Schritt 100
- 110: Schritt 110
- 120: Schritt 120
- 130: Schritt 130
- 140: Schritt 140
- 150: Schritt 150

## Patentansprüche

1. Verfahren zur Bestrahlung eines sich bewegenden Zielvolumens (14) in einem nicht-lebenden Körper oder zur Simulation einer Bestrahlung eines sich bewegenden Zielvolumens mit folgenden Schritten:
- Bestrahlen des Zielvolumens (14) derart, dass der Strahl (12), mit dem das Zielvolumen (14) bestrahlt wird, einer Bewegung des Zielvolumens (14) nachgeführt wird,
- Ermitteln einer Dosisbelegung, die in einem von dem Zielvolumen (14) unterschiedlichen Bereich (40, 42, 54, 62, 64, 66) bei der Bestrahlung des Zielvolumens (14) aufgetreten ist, wobei der Bereich (40, 42, 54, 62, 64, 66) einem anderen Bewegungsmuster als das Zielvolumen (14) unterliegt und/oder wobei der Bereich (40, 42, 54, 62, 64, 66) einer anderen bewegungsbedingten Partikelreichweitenänderung als das Zielvolumen unterliegt, **gekennzeichnet durch**:
- Berücksichtigen der Dosisbelegung, die in dem von dem Zielvolumen unterschiedlichen Bereichs bei der Bestrahlung des Zielvolumens aufgetreten ist zur Überprüfung der Bewegungsnachführung.

2. Verfahren nach Anspruch 1, wobei
bei Ermitteln der Dosisbelegung eine Dosisberechnung durchgeführt wird, mit der eine Dosis bestimmt wird, die in dem Bereich (40, 42, 54, 62, 64, 66) deponiert wird, wobei die Dosisberechnung unter Verwendung der während der Bestrahlung durchgeführten Bewegungsnachführung erfolgt.

3. Verfahren nach Anspruch 1 oder 2, wobei
als Dosisbelegung ein Bestrahlungsmuster (68) ermittelt wird, das in dem Bereich (40, 42, 54, 62, 64, 66) durch die Bestrahlung hervorgerufen wird.

4. Verfahren nach einem der Ansprüche 1 bis 3, wobei
die Dosisbelegung, die während der Bestrahlung in dem Bereich (40, 42, 54, 62, 64, 66) auftritt, mithilfe einer Strahlungsdetektionsvorrichtung (40, 42, 62, 64, 66) ermittelt wird.

5. Verfahren nach einem der vorstehenden Ansprüche, wobei
die Bewegungsnachführung dadurch überprüft wird, indem die ermittelte Dosisbelegung mit einer zu erwartenden Dosisbelegung verglichen wird.

6. Verfahren nach einem der Ansprüche 1 bis 5, wobei eine nachfolgende Bestrahlung des Zielvolumens (14) bzw. eine Bestrahlungsplanung für das Zielvolumen (14) anhand der ermittelten Dosisbelegung modifiziert wird, insbesondere durch eine Änderung der Bestrahlungsplanung und/oder durch einen Wechsel eines Bestrahlungsverfahrens.

7. Bestrahlungsanlage zum Bestrahlen eines sich bewegenden Zielvolumens (14)
- mit einer Bestrahlungsvorrichtung (10), die ausgebildet ist zum Bestrahlen des Zielvolumens (14) derart, dass bei einer Bestrahlung der Strahl (12) der Bewegung des Zielvolumens (14) nachgeführt wird,
- mit einer Überwachungsvorrichtung (58), die ausgebildet ist zum Ermitteln einer Dosisbelegung, die in einem von dem Zielvolumen (14) unterschiedlichen Bereich (40, 42, 54, 62, 64, 66) bei der Bestrahlung des Zielvolumens (14) auftritt, wobei der Bereich (40, 42, 54, 62, 64, 66) dadurch charakterisiert ist, dass der Bereich einem anderen Bewegungsmuster und/oder einer anderen bewegungsbedingten Partikelreichweitenänderung als das Zielvolumen (14) unterliegt,
**dadurch gekennzeichnet , dass** die Überwachungsvorrichtung (58) ferner dazu ausgebildet ist, die ermittelte Dosisbelegung zur Überprüfung der Bewegungsnachführung zu verwenden.

8. Bestrahlungsanlage nach Anspruch 7, wobei die Überwachungsvorrichtung ausgebildet ist zum Durchführen einer Dosisberechnung, mit der eine Dosis bestimmt wird, die bei der Bestrahlung in dem Bereich (40, 42, 54, 62, 64, 66) deponiert wird, und wobei die Dosisberechnung unter Verwendung der tatsächlich während der Bestrahlung durchgeführten Bewegungsnachführung erfolgt.

9. Bestrahlungsanlage nach Anspruch 7 oder 8, wobei die Überwachungsvorrichtung ausgebildet ist zum Ermitteln eines Bestrahlungsmusters (68), das in dem Bereich (40, 42, 54, 62, 64, 66) durch die Bestrahlung hervorgerufen wird.

10. Bestrahlungsanlage nach einem der Ansprüche 7 bis 9, wobei die Überwachungsvorrichtung (58) eine Strahlungsdetektionsvorrichtung (40, 42, 62, 64, 66) umfasst, mit der die Dosisbelegung, die während einer Bestrahlung in dem Bereich (40, 42, 54, 62, 64, 66) auftritt, ermittelbar ist.

11. Bestrahlungsanlage nach einem der Ansprüche 8 bis 10, wobei die Bestrahlungsanlage eine Bestrahlungsteuerungsvorrichtung (36, 44) umfasst, die ausgebildet ist zum Modifizieren einer nachfolgenden Bestrahlung des Zielvolumens (14) oder eine Änderung einer Bestrahlungsplanung für das Zielvolumen (14) unter Verwendung der ermittelten Dosisbelegung, insbesondere durch eine Änderung der Bestrahlungsplanung und/oder durch einen Wechsel des Bestrahlungsverfahrens.

12. Bestrahlungsanlage nach einem der Ansprüche 8 bis 11, wobei die Bestrahlungsanlage eine Erfassungseinrichtung (56) aufweist zum Aufzeichnen von Daten, mit denen eine während einer Bestrahlung durchgeführte Bewegungsnachführung ermittelbar ist.

## Claims

1. A method for irradiation of a moving target volume (14) in a non-living body or for simulating irradiation of a moving target volume, comprising the steps of:
- irradiating the target volume (14) in a manner so that the beam (12) used to irradiate the target volume (14) tracks the movement of the target volume (14);
- determining a dose distribution that occurred in a region (40, 42, 54, 62, 64, 66) different from the target volume (14) during the irradiation of the target volume (14), wherein said region (40, 42, 54, 62, 64, 66) is subject to a different movement pattern than the target volume (14) and/or wherein said region (40, 42, 54, 62, 64, 66) is subject to a different motion-related change in particle range than the target volume,
**characterized by**:
- taking into account the dose distribution that occurred in the region different from the target volume during the irradiation of the target volume for checking the motion tracking.

2. The method according to claim 1, wherein when determining the dose distribution a dose calculation is performed to determine a dose that is deposited in said region (40, 42, 54, 62, 64, 66), wherein the dose calculation is carried out using the motion tracking performed during the irradiation.

3. The method according to claim 1 or 2, wherein the dose distribution is determined as an irradiation pattern (68) caused in said region (40, 42, 54, 62, 64, 66) by the irradiation.

4. The method according to any of claims 1 to 3, wherein the dose distribution occurring in said region (40, 42, 54, 62, 64, 66) during the irradiation is determined using a radiation detection device (40, 42, 62, 64, 66).

5. The method according to any of the preceding claims, wherein the motion tracking is checked by comparing the determined dose distribution with an expected dose distribution.

6. The method according to any of claims 1 to 5, wherein a subsequent irradiation of the target volume (14) or an irradiation planning for the target volume (14) is modified based on the determined dose distribution, in particular by changing the irradiation planning and/or by changing the irradiation method.

7. An irradiation system for irradiating a moving target volume (14), comprising:
- an irradiation device (10) adapted to irradiate the target volume (14) in such a manner that the beam (12) tracks the movement of the target volume (14) during an irradiation;
- a monitoring device (58) adapted to determine a dose distribution occurring in a region (40, 42, 54, 62, 64, 66) different from the target volume (14) during the irradiation of the target volume (14), wherein said region (40, 42, 54, 62, 64, 66) is distinguished by being subject to a different movement pattern and/or a different motion-related change in particle range than the target volume (14);
**characterized in that** said monitoring device (58) is further adapted to use the determined dose distribution for checking the motion tracking.

8. The irradiation system according to claim 7, wherein the monitoring device is adapted to perform a dose calculation by which a dose is determined which is deposited in said region (40, 42, 54, 62, 64, 66) during the irradiation, and wherein the dose calculation is carried out using the motion tracking actually performed during the irradiation.

9. The irradiation system according to claim 7 or 8, wherein the monitoring device is adapted to determine an irradiation pattern (68) caused in said region (40, 42, 54, 62, 64, 66) by the irradiation.

10. The irradiation system according to any of claims 7 to 9, wherein the monitoring device (58) comprises a radiation detection device (40, 42, 62, 64, 66) by means of which the dose distribution occurring in said region (40, 42, 54, 62, 64, 66) during an irradiation can be determined.

11. The irradiation system according to any of claims 8 to 10, wherein the irradiation system comprises an irradiation control device (36, 44) adapted for modifying a subsequent irradiation of the target volume (14) or changing an irradiation planning for the target volume (14) using the determined dose distribution, in particular by changing the irradiation planning and/or changing the irradiation method.

12. The irradiation system according to any of claims 8 to 11, wherein the irradiation system comprises an acquisition device (56) for acquiring data from which a motion tracking performed during an irradiation can be determined.

## Revendications

1. Procédé d'irradiation d'un volume cible (14) en mouvement dans un corps non vivant ou de simulation d'une irradiation d'un volume cible en mouvement, comportant les étapes suivantes :
- irradiation du volume cible (14) de façon que le faisceau (12) avec lequel le volume cible (14) est irradié suive un mouvement du volume cible (14),
- détermination d'une distribution de dose survenue dans une zone (40, 42, 54, 62, 64, 66) différente du volume cible (14) lors de l'irradiation du volume cible (14), cette zone (40, 42, 54, 62, 64, 66) étant soumise à un autre modèle de mouvement que le volume cible (14) et/ou la zone (40, 42, 54, 62, 64, 66) étant soumise à une autre variation de portée des particules due au mouvement que le volume cible, **caractérisé par** l'étape suivants :
- prise en compte de la distribution de dose survenue dans la zone différente du volume cible lors de l'irradiation du volume cible pour vérifier le suivi de mouvement.

2. Procédé selon la revendication 1, selon lequel, lors de la détermination de la distribution de dose est effectué un calcul de dose qui sert à déterminer une dose déposée dans la zone (40, 42, 54, 62, 64, 66), le calcul de dose étant réalisé en utilisant le suivi de mouvement effectué pendant l'irradiation.

3. Procédé selon la revendication 1 ou 2, selon lequel la distribution de dose déterminée est un modèle d'irradiation (68) provoqué par l'irradiation dans la zone (40, 42, 54, 62, 64, 66).

4. Procédé selon l'une des revendications 1 à 3, selon lequel la distribution de dose survenant lors de l'irradiation dans la zone (40, 42, 54, 62, 64, 66) est déterminée à l'aide d'un dispositif de détection de rayonnement (40, 42, 62, 64, 66).

5. Procédé selon l'une des revendications précédentes, selon lequel le suivi de mouvement est vérifié en comparant la distribution de dose déterminée avec une distribution de dose attendue.

6. Procédé selon l'une des revendications 1 à 5, selon lequel une irradiation ultérieure du volume cible (14) ou un plan d'irradiation pour le volume cible (14) est modifié à l'aide de la distribution de dose déterminée, en particulier par une modification du plan d'irradiation et/ou par un changement de procédé d'irradiation.

7. Installation d'irradiation conçue pour irradier un volume cible (14) en mouvement
- comprenant un dispositif d'irradiation (10) conçu pour irradier le volume cible (14) de façon que, lors d'une irradiation, le faisceau (12) suive le mouvement du volume cible (14),
- comprenant un dispositif de surveillance (58) conçu pour déterminer une distribution de dose survenant dans une zone (40, 42, 54, 62, 64, 66) différente du volume cible (14) lors de l'irradiation du volume cible (14), la zone (40, 42, 54, 62, 64, 66) étant **caractérisée en ce que** la zone est soumise à un autre modèle de mouvement et/ou à une autre variation de portée des particules due au mouvement que le volume cible (14),
**caractérisée en ce que** le dispositif de surveillance (58) est en outre conçu pour utiliser la distribution de dose déterminée pour vérifier le suivi de mouvement.

8. Installation d'irradiation selon la revendication 7, dans laquelle le dispositif de surveillance est conçu pour effectuer un calcul de dose servant à déterminer une dose qui est déposée dans la zone (40, 42, 54, 62, 64, 66) lors de l'irradiation, et dans laquelle le calcul de dose est réalisé en utilisant le suivi de mouvement effectivement effectué pendant l'irradiation.

9. Installation d'irradiation selon la revendication 7 ou 8, dans laquelle le dispositif de surveillance est conçu pour déterminer un modèle d'irradiation (68) provoqué par l'irradiation dans la zone (40, 42, 54, 62, 64, 66).

10. Installation d'irradiation selon l'une des revendications 7 à 9, dans laquelle le dispositif de surveillance (58) comprend un dispositif de détection de rayonnement (40, 42, 62, 64, 66) qui permet de déterminer la distribution de dose survenant dans la zone (40, 42, 54, 62, 64, 66) pendant une irradiation.

11. Installation d'irradiation selon l'une des revendications 8 à 10, dans laquelle l'installation d'irradiation comprend un dispositif de commande d'irradiation (36, 44) conçu pour modifier une irradiation ultérieure du volume cible (14) ou un plan d'irradiation pour le volume cible (14) en utilisant la distribution de dose déterminée, en particulier par une modification du plan d'irradiation et/ou par un changement de procédé d'irradiation.

12. Installation d'irradiation selon l'une des revendications 8 à 11, dans laquelle l'installation d'irradiation comporte un dispositif de détection (56) pour enregistrer des données permettant de déterminer un suivi de mouvement effectué pendant une irradiation.
